(19) Europäisches Patentamt
European Patent Office
Office européen des brevets

(11) EP 4 650 342 A1

(12) EUROPEAN PATENT APPLICATION
published in accordance with Art. 153(4) EPC

(43) Date of publication:
**19.11.2025 Bulletin 2025/47**

(21) Application number: **24875667.8**

(22) Date of filing: **16.04.2024**

(51) International Patent Classification (IPC):
***C07C 217/90*** *(2006.01)*

(86) International application number:
**PCT/CN2024/087918**

(87) International publication number:
**WO 2025/208664 (09.10.2025 Gazette 2025/41)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **01.04.2024 CN 202410383942**

(71) Applicant: **Yifan Biotechnology (Shanghai) Co., Ltd.**
**Shanghai 200050 (CN)**

(72) Inventors:
- **WANG, Pandeng**
  **Shanghai 200050 (CN)**
- **WU, Haiqin**
  **Shanghai 200050 (CN)**
- **WU, Kechong**
  **Shanghai 200050 (CN)**
- **JIA, Junchao**
  **Shanghai 200050 (CN)**
- **GUO, Kaijie**
  **Shanghai 200050 (CN)**
- **WANG, Jia**
  **Shanghai 200050 (CN)**

(74) Representative: **Bayramoglu et al.**
**Mira Office**
**Kanuni Sultan Süleyman Boulevard 5387**
**Street Beytepe, floor 12, no:50**
**06800 Cankaya, Ankara (TR)**

# (54) CRYSTAL FORM OF ACLONIFEN, AND PREPARATION METHOD THEREFOR AND USE THEREOF

(57) The present disclosure provides a crystal form of aclonifen and a preparation method and use of the crystal form. In the present disclosure, the crystal form of aclonifen is prepared through the recrystallization of amorphous aclonifen, and is called an aclonifen solid form A. In the present disclosure, the crystal is analyzed by various analytical methods, and the aclonifen solid form A is used to prepare a stable agricultural chemical preparation. The preparation method of the aclonifen solid form A provided by the present disclosure has a high recovery rate, is simple, and can be scaled up. The crystal form of the aclonifen solid form A exhibits high stability when used in a preparation, and has a promising application prospect.

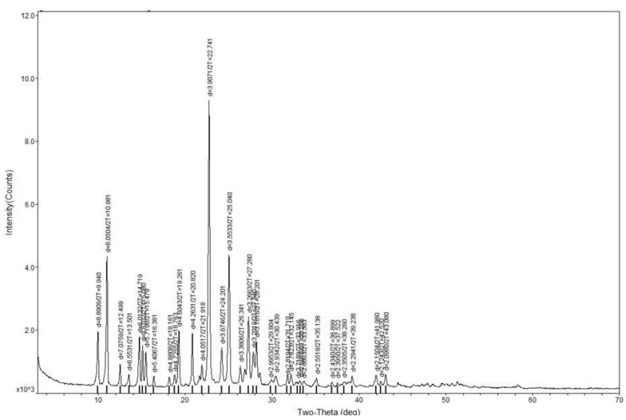


FIG. 2

EP 4 650 342 A1

## Description

### TECHNICAL FIELD

The present disclosure relates to a crystal form of aclonifen and a preparation method and use of the crystal form, and belongs to the technical field of agricultural herbicides.

### BACKGROUND TECHNOLOGY

Aclonifen is a diphenyl ether herbicide and is an inhibitor for protoporphyrinogen oxidase. When applied before sprouting, aclonifen can control broadleaf weeds and grassy weeds in the sunflower, potato, and winter wheat fields. Aclonifen exhibits an excellent control effect for *Alopecurus aequalis, Eragrostis ferruginea,* etc., in pea and carrot seedling fields. Due to advantages such as relative high efficiency, low toxicity, and low residues, aclonifen occupies an important position in the pesticide industry and is widely used.

Aclonifen and the method for preparing aclonifen are known in the art. It is also known that aclonifen has a weed control effect. The industrial synthesis method with a reasonable cost for an aclonifen active ingredient is as follows: 1,2,3-trichlorobenzene as the raw material is nitrified to produce 2,3,4-trichloronitrobenzene; then 2,3,4-trichloronitrobenzene is allowed to react with ammonia to produce an ammonolysis product, 2,3-dichloro-6-nitroaniline; and then 2,3-dichloro-6-nitroaniline is subjected to etherification with phenol or sodium phenolate to produce aclonifen. References: For example, in the patent US4394159, 2,3-dichloro-6-nitroaniline is allowed to react with phenol or sodium phenolate; the yield is 74%, and a synthesis route is as follows:

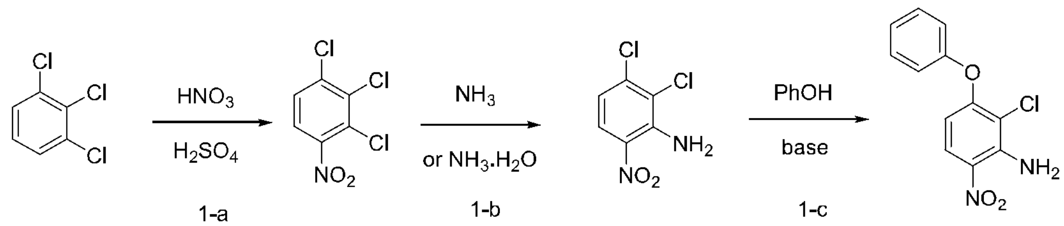


Among the current published reports on aclonifen, there are no reports on crystal forms of aclonifen and data related to the crystal forms. It is well known in the field of pesticides that different crystal forms of a pesticide active ingredient bring different effects, and economically important preparations of a pesticide active ingredient may also have different properties. Moreover, it has been found that aclonifen prepared by the above-known method exists in an amorphous form. Similarly, it is impossible to acquire a resolvable X-ray powder diffraction (X-RPD) pattern for aclonifen prepared by the above-known method, as shown in FIG. 1.

Further, aclonifen in an amorphous state has relatively poor storage stability and is prone to aggregation, especially after long-term storage. Thus, it is not suitable for the preparation of aclonifen in an amorphous state into compositions or preparations. Such economically important preparations include, for example, granules, encapsulated granules (GRs), tablets, water-dispersible granules (WGs), water-dispersible tablets, water-dispersible powders, etc. In some important preparations, an active compound exists in a dispersed form, such as suspension concentrates (SCs). The preparations are economically relevant and should have excellent storage stability. Therefore, there is a need to develop a novel solid form with storage stability for aclonifen.

### CONTENT OF THE INVENTION

An objective of the present disclosure is to solve the technical problem that the existing aclonifen in an amorphous form has poor storage stability, is prone to aggregation after storage, and is not suitable for the preparation into a composition or preparation, and the present disclosure provides a crystal form of aclonifen and a preparation method and use of the crystal form.

To achieve the above objective, the present disclosure adopts the following technical solutions:
A first aspect of the present disclosure provides a crystal form of aclonifen, where the crystal form is denoted as an aclonifen solid form A; and the crystal form presents the following reflections at $2\theta\pm0.20°$ in any combination in an X-RPD pattern recorded at 25°C using Cu-Ka radiation:

$$2\theta = 9.94\pm0.20 \qquad (1)$$

$$2\theta = 10.98 \pm 0.20 \quad (2)$$

$$2\theta = 14.72 \pm 0.20 \quad (3)$$

$$2\theta = 15.08 \pm 0.20 \quad (4)$$

$$2\theta = 15.48 \pm 0.20 \quad (5)$$

$$2\theta = 19.26 \pm 0.20 \quad (6)$$

$$2\theta = 20.82 \pm 0.20 \quad (7)$$

$$2\theta = 22.74 \pm 0.20 \quad (8)$$

$$2\theta = 24.20 \pm 0.20 \quad (9)$$

$$2\theta = 25.04 \pm 0.20 \quad (10)$$

$$2\theta = 27.28 \pm 0.20 \quad (11)$$

$$2\theta = 28.20 \pm 0.20 \quad (12).$$

Preferably, the aclonifen solid form A has an infrared (IR) spectrum with a characteristic functional group vibration peak at one or more wavenumbers ($cm^{-1}$, ±0.2%) of 3,503 $cm^{-1}$, 3,390 $cm^{-1}$, 1,621 $cm^{-1}$, 1,591 $cm^{-1}$, 1,568 $cm^{-1}$, 1,551 $cm^{-1}$, 1,485 $cm^{-1}$, 1,462 $cm^{-1}$, 1,421 $cm^{-1}$, 1,357 $cm^{-1}$, 1,243 $cm^{-1}$, 1,112 $cm^{-1}$, and 754 $cm^{-1}$; and/or
the aclonifen solid form A has a differential scanning calorimetry (DSC) curve with an endothermic melting peak at 82.5°C. The aclonifen solid form A preferably has an enthalpy of fusion of 72 J/g.

Preferably, the aclonifen solid form A is characterized by an X-RPD pattern essentially as shown in FIG. 2, and/or by an IR spectrum essentially as shown in FIG. 3, and/or by a DSC curve essentially as shown in FIG. 4.

The aclonifen solid form A exhibits significantly-improved storage stability, which can significantly solve the problem that the current commercially-available aclonifen preparations have poor stability during long-term storage, especially the problem that SC dosage forms have poor stability during long-term storage. In addition, it has been found that, compared with the amorphous aclonifen prepared according to the disclosure in the patent US4394159, the aclonifen solid form A can exhibit high stability when used in a preparation. Further, due to excellent thermodynamic stability, the aclonifen solid form A will not be transformed into another form even after long-term storage, which allows the economically-relevant applications and can provide a desired long storage period for preparations.

A method for preparing amorphous aclonifen is well known in the art. The amorphous aclonifen is manufactured on a commercial scale and is available. A particularly suitable method for preparing amorphous aclonifen is described in the patent US4394159.

A second aspect of the present disclosure provides a method for preparing the aclonifen solid form A described in the first aspect of the present disclosure, including the following steps:

step 1, dissolving aclonifen in a solvent, where the aclonifen is amorphous aclonifen;
step 2, precipitating dissolved aclonifen to produce the aclonifen solid form A; and
step 3, separating the precipitated aclonifen solid form A.

Preferably, in the step 1, the solvent is selected from any of the following solvents: methanol, ethanol, ethyl acetate, toluene, xylene, chlorobenzene, or any combination thereof, or a mixture of toluene and n-hexane, a mixture of toluene and cyclohexane, a mixture of ethyl acetate and n-hexane, a mixture of dichloromethane and n-hexane, a mixture of tetrahydrofuran (THF) and water, a mixture of N,N-dimethylformamide (DMF) and water, a mixture of methanol and

water, and a mixture of ethanol and water.

Preferably, the solvent is selected from at least one of methanol and ethanol.

Preferably, in the step 1, the aclonifen is dissolved in the solvent by heating.

Preferably, in the step 1, the aclonifen is dissolved in the solvent by heating from room temperature to or below a refluxing temperature for the solvent.

Preferably, the heating is conducted to 60°C to 90°C.

Preferably, a solution prepared in the step 1 is then cooled to a temperature of about 0°C to 15°C to crystallize a desired crystal form from the solvent. The aclonifen solid form A can also be crystallized as follows: a specified amount of the solvent is removed by cooling to below the refluxing temperature for the solvent or the solvent mixture under or not under vacuum to concentrate the homogeneous solution.

Preferably, the aclonifen solid form A can also be produced as follows: a crystal seed for the desired crystal form (which can facilitate or accelerate the crystallization) is added to the solution prepared in the step 1 during crystallization. A weight of the crystal seed added to the solution is usually 0.001% to 5% and further preferably 0.005% to 1.0% of a weight of the solution produced after the aclonifen is dissolved in the solvent in the step 1. Optionally, at a temperature below a boiling point of a corresponding solvent or solvent mixture, the crystal seed is added to a concentrated solution.

Preferably, the precipitated aclonifen solid form A obtained in the step 2 is separated from a solution by a common solid component separation technique (such as filtration, centrifugation, or decantation). The separated solid is then washed one or more times with a solvent. Preferably, the solvent adopted for the washing can be the solvent adopted for the dissolution in the step 1 described above. According to the solubility of the crystal, the washing is usually conducted at 0°C to room temperature with a corresponding solvent to minimize the loss of crystalline substances in the corresponding washing solvent.

Preferably, the aclonifen solid form A is prepared through dissolution and recrystallization.

Preferably, in the step 2, the precipitating dissolved aclonifen to produce the aclonifen solid form A specifically includes: concentrating a solution and/or cooling and/or adding a solvent for reducing solubility and/or adding a crystal seed of the aclonifen solid form A.

Preferably, in the step 2, the dissolved aclonifen is precipitated into the aclonifen solid form A through gradient cooling, where it needs to be finally cooled to 0°C to 15°C for crystal precipitation.

A third aspect of the present disclosure provides an aclonifen solid form A prepared by the method described in the second aspect of the present disclosure, where the aclonifen solid form A has an aclonifen solid form A content of at least 97% in a weight percentage.

A fourth aspect of the present disclosure provides a composition, including the aclonifen solid form A described in the first aspect or the third aspect of the present disclosure and at least one additive, where the additive is selected from one or more of the following: a surfactant, a diluent, a dispersing agent, a wetting agent, an antioxidant, a defoaming agent, an antifreeze agent, and a thickening agent.

Preferably, the composition is in the following form: SC, a soluble liquid (SL), a dispersible concentrate (DC), an emulsifiable concentrate (EC), an emulsion seed-dressing agent, a granule (GR), a suspoemulsion (SE), an oil-based dispersion (OD), a soluble granule (SG), a microgranule (MG), or WG.

Preferably, the composition is in a form of SC.

Preferably, the composition includes the aclonifen solid form A of less than 80% in a weight percentage.

Preferably, the composition includes the aclonifen solid form A of 60% in a weight percentage.

The use of aclonifen as an herbicide is well known in the art, and aclonifen is used on a commercial scale. The aclonifen solid form A is also active in controlling noxious weeds. Therefore, techniques regarding the preparation of amorphous aclonifen and the application of aclonifen known in the art (such as those disclosed in the prior art literature described above) can also be applied in a similar manner to the aclonifen solid form A of the present disclosure.

Accordingly, the present disclosure provides an herbicide composition including the aclonifen solid form A as defined above.

In the present disclosure, the aclonifen solid form A can exist at a concentration sufficient to achieve a required dose when applied to a plant or a site of the plant, and it is expected that the aclonifen solid form A exists at a concentration of about 1% to about 80% based on a total weight of a mixture. For example, water, a solvent, and a carrier are added to the aclonifen solid form A, and if appropriate, an emulsifier and/or a dispersing agent and/or another additive is/are used to prepare preparations.

These preparations are prepared by mixing the aclonifen solid form A with at least one additive (such as a surfactant, a diluent, a wetting agent, a dispersing agent, a thickening agent, an antifreeze agent, a defoaming agent, an antioxidant, and any necessary adjuvant, and other preparation components).

The surfactant can be an ionic or non-ionic emulsifier, dispersing agent, or wetting agent. Accessible examples include, but are not limited to, a salt of polyacrylic acid, a lignosulfonate salt, a salt of benzenesulfonic acid or naphthalenesulfonic acid, a condensation polymer of epoxyethane with a fatty alcohol or a fatty acid or a fatty amine, a substituted phenol (especially an alkylphenol), a sulfosuccinate salt, a taurine derivative (especially alkyl taurate), or a

phosphate ester of a polyethoxylated phenol or alcohol. Preferably, the surfactant is selected from lignosulfonate salts (such as calcium lignosulfonate).

The diluent includes, but is not limited to, water, DMF, ethylene glycol, polypropylene glycol, propylene carbonate, a dibasic ester, paraffin, alkylbenzene, alkyl naphthalene, glycerin, olive oil, castor oil, linseed oil, sesame oil, corn oil, peanut oil, cottonseed oil, soybean oil, rapeseed oil, and coconut oil, ketones (such as cyclohexanone, 2-heptanone, and 4-hydroxy-4-methyl-2-pentanone), acetate esters (such as hexyl acetate, heptyl acetate, and octyl acetate), water, and alcohols (such as methanol, cyclohexanol, decyl alcohol, benzyl alcohol, and tetrahydrofurfuryl alcohol), salts such as alkali metal phosphates (such as sodium dihydrogen phosphate), alkaline earth metal phosphates, sulfates of sodium, potassium, magnesium, and zinc, sodium chloride, potassium chloride, sodium acetate, sodium carbonate, and sugar and sugar derivatives such as sorbitol, lactose, sucrose, and mannitol, clay, synthetic silica, and diatomaceous earth, calcium silicate, titanium dioxide, alumina, calcium oxide, and zinc oxide, and a mixture thereof.

The wetting agent includes, but is not limited to, a phosphate ester, an alkynediol, an ethoxylated silicone, sodium dodecyl sulfate, Nekal, a Chinese honey locust powder, a detergent LS (sodium p-methoxyfattyamidobenzene sulfonate), an alkyl-substituted naphthalene sulfonate ester, and a polyalkylene glycol ether. Preferably, the wetting agent is selected from a polyalkylene glycol ether, sodium dodecyl sulfate, and a Chinese honey locust powder.

The dispersing agent includes, but is not limited to: a polycarboxylate salt, a sulfate salt of an alkylphenol ethoxylate-formaldehyde condensate, calcium alkyl benzene sulfonate, an alkylphenol ethoxylate, a fatty amine polyoxyethylene ether, a polyoxyethylene fatty acid, a glycerol fatty acid polyoxyethylene ether, sodium, calcium, and ammonium salts of lignosulfonic acid, and a naphthalene sulfonate-formaldehyde condensate. Preferably, the dispersing agent is selected from lignosulfonate salts (such as calcium lignosulfonate) and calcium alkyl benzene sulfonate.

The thickening agent includes, but is not limited to, guar gum, pectin, casein, carrageenan, xanthan gum, an alginate salt, methylcellulose, hydroxyethylcellulose, hydroxypropylcellulose, and carboxymethylcellulose. Appropriate thickening agents include derivatives of the above categories; polyvinyl alcohol, polyacrylamide, polyvinylpyrrolidone, and various polyethers, and copolymers thereof; and polyacrylic acid and salts thereof. A preferred thickening agent is selected from xanthan gum and polyvinyl alcohol.

An appropriate antifreeze agent is a liquid polyol, such as ethylene glycol, propylene glycol, or glycerin. A weight of the antifreeze agent is usually about 1% to about 20% and particularly about 5% to 10% of a total weight of the composition.

The defoaming agent includes all substances that can generally be used for this purpose in agricultural chemical compositions. An appropriate defoaming agent is known in the art, and is commercially available. A preferred defoaming agent is a mixture of polydimethylsiloxane and perfluoroalkyl phosphonic acid or a polyether-modified silicone.

The antioxidant includes all substances that are known in the art and can generally be used for this purpose in agricultural chemical compositions. Preferably, the antioxidant is butylated hydroxyanisole (BHA) and butylated hydroxytoluene (BHT).

A pH-regulating agent includes all substances that are known in the art and can generally be used for this purpose in agricultural chemical compositions. Preferably, the pH-regulating agent is citric acid and tartaric acid.

Other preparation components may also be used in the present disclosure, such as a preservative and a permeating agent. An herbicide can also be added to the composition including the aclonifen solid form A in the present disclosure. An appropriate herbicide combination, such as the addition of one or more herbicide components at an appropriate amount, may lead to an improved combined weeding effect. These components are known to those skilled in the art.

A fifth aspect of the present disclosure provides a use of the aclonifen solid form A described in the first or third aspect of the present disclosure or the composition described in the fourth aspect of the present disclosure in preparation of an herbicide.

The herbicide can be used to control noxious weeds, and the noxious weeds are selected from grassy weeds and broadleaf weeds.

Preferably, the noxious weeds are selected from *Alopecurus aequalis, Eragrostis ferruginea,* bedstraw, *Lamium barbatum, Myosotis arvensis,* chickweed, ivy, *Veronica polita, Veronica undulata,* and *Viola arvensis Murray.*

Preferably, the herbicide is provided to control noxious weeds in ornamental plants, fruit trees, vegetables, legume crops, and cereal crops.

Preferably, the herbicide is provided to control noxious weeds in winter wheat, potato, sunflower, sugarbeet, sugarcane, carrot, corn, and soybean.

The present disclosure also provides a method for controlling a weed, including: applying a weeding-effective amount of the aclonifen solid form A described above or the composition described above to a plant, a part of the plant, or an environment around the plant. Accordingly, the present disclosure also provides a method for controlling a weed in a plant, a part of the plant, and/or an environment around the plant, including: applying an effective amount of the aclonifen solid form A or the composition to a root of the plant, the part of the plant, or the environment around the plant.

When used in conjunction with a numerical value or range, the term "about" used herein means that it is slightly

greater or less than the numerical value or range, and deviates from an endpoint of the numerical value or range by ±10%.

The treatment of a plant and a part of the plant with the composition or preparation of the present disclosure refers to allowing the composition or preparation to act on a surrounding environment, a habitat, or a storage space for the plant and the part of the plant directly or by a conventional treatment method. Examples of the conventional treatment method include impregnation, spraying, vaporization, atomization, spreading, brush-coating, mixing (such as a soil treatment), etc.

The term "room temperature" used herein refers to a temperature range of about 20°C to 25°C.

In general, the crystalline material can be identified as follows: for example, a diffraction peak is produced under X-ray radiation and/or an endothermic melting peak curve with a characteristic peak is presented in DSC. Unless otherwise stated, all percentages in the present disclosure refer to wt%.

Compared with the prior art, the present disclosure has the following beneficial effects:

1. The present disclosure provides for the first time a novel aclonifen crystal form named "solid form A". The present disclosure also provides an X-RPD pattern, an IR spectrum, a DSC curve, an enthalpy of fusion, etc. of the aclonifen solid form A.
2. The aclonifen solid form A can significantly solve the problem that the current aclonifen preparations, especially SC dosage forms of aclonifen, have poor stability during long-term storage. The aclonifen solid form A can exhibit high stability when used in a preparation. Further, due to excellent thermodynamic stability, the aclonifen solid form A will not be transformed into another form even after long-term storage, which allows the economically-relevant applications and can provide a desired long storage period for preparations.
3. The present disclosure provides a method for preparing the aclonifen solid form A. The method involves a simple preparation flow and simple operations, and allows a high recovery rate, a high comprehensive utilization rate of a raw material, and a high content.

## DESCRIPTION OF THE DRAWINGS

FIG. 1 shows an X-RPD pattern of amorphous aclonifen;
FIG. 2 shows an X-RPD pattern of an aclonifen solid form A;
FIG. 3 shows an IR spectrum of the aclonifen solid form A; and
FIG. 4 shows a DSC curve of the aclonifen solid form A.

## SPECIFIC IMPLEMENTATIONS

In order to make the present disclosure more understandable, the preferred examples are provided to describe the present disclosure in detail below with reference to the accompanying drawings.

In the following examples of the present disclosure, a powder diffractometer was used to acquire each X-RPD pattern at 25°C under the following acquisition parameters:

| |
|---|
| Ultima IV X-ray diffractometer |
| θ-compensating slit and graphite monochromator |
| Copper (K-a) radiation, 40 kV, 40 mA |
| Step size: 0.02° 2-θ |
| Counting time: 1.0 s |
| Maximum peak intensity: 9,285 counts/s |
| Scan range: 3°-70° 2-θ |

Example 1 Preparation of amorphous aclonifen

The amorphous aclonifen was prepared according to the disclosure in the patent US4394159. The method for preparing aclonifen from purified 2,3-dichloro-6-nitroaniline in Example 22 (b-ii) was appropriately modified. There are several methods for preparing aclonifen reported in the prior art. Thus, the amorphous aclonifen can be manufactured on a commercial scale with one of the reported methods or can be purchased in bulk through various manufacturing industries.

The aclonifen can be conveniently prepared with purified 2,3-dichloro-6-nitroaniline and phenol by one of the processes reported in the patent US4394159.

15.5 g of purified 2,3-dichloro-6-nitroaniline, 75 mL of dimethyl sulfoxide, and 7.8 g of phenol were added successively to a reaction bottle, and slowly stirred. At room temperature, 11.5 g of potassium carbonate was added. A temperature was raised to 50°C, and stirring was conducted to allow a reaction for 8 h. After the reaction was completed, a resulting mixture was acidified with 10 g of glacial acetic acid. A resulting product was precipitated with 400 g of ice water, filtered, and dried to produce 19.5 g of a yellow solid, with a yield of 98%. The aclonifen had a melting point of 80.5°C.

As shown in FIG. 1, an X-RPD pattern of the aclonifen product did not have a significant signal, indicating that the aclonifen product prepared according to the disclosure of the patent US4394159 was amorphous.

Example 2 Preparation of an aclonifen solid form A

15 g of the amorphous aclonifen sample prepared in Example 1 and 150 mL of methanol were placed together in a three-neck round-bottom flask, and a resulting slurry was heated to 65°C to produce a homogeneous solution. The homogeneous solution was stirred at 65°C for 1 h, and insoluble particles (if any) were filtered out. A resulting solution was slowly and uniformly cooled to 55±2°C within 2 h and subjected to heat preservation for 0.5 h, then slowly and uniformly cooled to 40±2°C within 2 h and subjected to heat preservation for 0.5 h, and then slowly cooled to 5±2°C within 2 h to produce a fine crystal. A resulting heterogeneous mixture was stirred at 5±2°C for 0.5 h. A resulting slurry was then filtered and washed with 10 mL of cold methanol. The separated crystal was dried at 60°C. The crystal product had a purity of higher than 97%, a mass of the crystal product was 14.0 g, and a yield of the crystal product was 93%.

The crystal was analyzed by X-RPD, IR spectroscopy, and DSC. The crystal was the aclonifen solid form A shown in FIG. 2, FIG. 3, and FIG. 4. An IR spectrum of the aclonifen solid form A was shown in FIG. 2. The IR spectrum of the aclonifen solid form A had a functional group characteristic vibration peak at one or more wavenumbers of 3,503 $cm^{-1}$, 3,390 $cm^{-1}$, 1,621 $cm^{-1}$, 1,591 $cm^{-1}$, 1,568 $cm^{-1}$, 1,551 $cm^{-1}$, 1,485 $cm^{-1}$, 1,462 $cm^{-1}$, 1,421 $cm^{-1}$, 1,357 $cm^{-1}$, 1,243 $cm^{-1}$, 1,112 $cm^{-1}$, and 754 $cm^{-1}$.

A DSC curve of the aclonifen solid form A presented an endothermic melting peak at 82.4°C and an enthalpy of fusion of 72 J/g, as shown in FIG. 4.

An X-RPD pattern of the crystal presented the reflections shown in FIG. 2, and the values were summarized in the following Table 1:

Table 1 Reflections in the X-RPD pattern of the aclonifen solid form A

| Aclonifen solid form A | |
|---|---|
| 2θ | d(Å) |
| 9.940±0.200 | 8.89±0.05 |
| 10.981±0.200 | 8.05±0.05 |
| 12.499±0.200 | 7.07±0.05 |
| 13.501±0.200 | 6.55±0.05 |
| 14.719±0.200 | 6.01±0.05 |
| 15.080±0.200 | 5.87±0.05 |
| 15.479±0.200 | 5.72±0.05 |
| 19.261±0.200 | 4.60±0.05 |
| 20.820±0.200 | 4.26±0.05 |
| 21.918±0.200 | 4.05±0.05 |
| 22.741±0.200 | 3.90±0.05 |
| 24.201±0.200 | 3.67±0.05 |
| 25.040±0.200 | 3.55±0.05 |
| 26.341±0.200 | 3.38±0.05 |
| 27.280±0.200 | 3.26±0.05 |
| 27.840±0.200 | 3.20±0.05 |
| 28.201±0.200 | 3.16±0.05 |

(continued)

| Aclonifen solid form A | |
|---|---|
| 2θ | d(Å) |
| 30.439±0.200 | 2.93±0.05 |
| 32.145±0.200 | 2.78±0.05 |
| 39.238±0.200 | 2.29±0.05 |
| 41.980±0.200 | 2.15±0.05 |
| 43.080±0.200 | 2.09±0.05 |

Example 3 Preparation of an aclonifen solid form A

15 g of the amorphous aclonifen sample prepared in Example 1 and 300 mL of ethanol were placed together in a three-neck round-bottom flask, and a resulting slurry was heated to 78°C to produce a homogeneous solution. The homogeneous solution was stirred at 78°C for 1 h, and insoluble particles (if any) were filtered out. A resulting solution was slowly and uniformly cooled to 25±2°C within 2 h. A resulting homogeneous mixture was poured into an open glass container and allowed to stand in a ventilated sample cabinet, such that the solvent was volatilized at room temperature to produce a fine crystal. A resulting heterogeneous mixture was stirred at 10±2°C for 0.5 h. A resulting slurry was then filtered and washed with 10 mL of cold ethanol. The separated crystal was dried at 60°C. The crystal product had a purity of higher than 97%, a mass of the crystal product was 13.2 g, and a yield of the crystal product was 88%.

Example 4 Preparation of SC of the amorphous aclonifen

All the components listed in Table 2 were thoroughly mixed and then ground with a grinder to produce the SC.

Table 2 Components for the SC of the amorphous aclonifen

| Content | wt% | Function |
|---|---|---|
| Amorphous aclonifen, 97% (prepared in Example 1) | 60.0 | Active ingredient |
| Calcium lignosulfonate | 5.0 | Surfactant |
| Polyalkylene glycol ether | 4.0 | Wetting agent |
| BHA | 0.5 | Antioxidant |
| Polydimethylsiloxane | 0.5 | Defoaming agent |
| Glycerin | 9.0 | Antifreeze agent |
| Xanthan gum | 0.3 | Thickening agent |
| Citric acid | 0.02 | pH-regulating agent |
| Water | Making up to 100 | Diluent |

Example 5 Preparation of SC of an aclonifen solid form A

All the components listed in Table 3 were thoroughly mixed and then ground with a grinder to produce the SC.

Table 3 Components for the SC of the aclonifen solid form A

| Content | wt% | Function |
|---|---|---|
| Aclonifen solid form A, 97% (prepared in Example 2) | 60.0 | Active ingredient |
| Calcium lignosulfonate | 5.0 | Surfactant |
| Polyalkylene glycol ether | 4.0 | Wetting agent |
| BHA | 0.5 | Antioxidant |
| Polydimethylsiloxane | 0.5 | Defoaming agent |

(continued)

| Content | wt% | Function |
|---|---|---|
| Glycerin | 9.0 | Antifreeze agent |
| Xanthan gum | 0.3 | Thickening agent |
| Citric acid | 0.02 | pH-regulating agent |
| Water | Making up to 100 | Diluent |

Example 6 Storage stability comparison

The samples prepared in Examples 4 and 5 each were stored in a heating oven with a same atmosphere at 54°C continuously for 1 month, 3 months, and 6 months. The procedure followed was carried out in accordance with CIPAC MT 46.3. A concentration of aclonifen was measured by high-performance liquid chromatography (HPLC) at the end of each storage time. The aggregation was measured through observation. The original concentration of aclonifen in each preparation was 60%. Results were listed in Table 4.

Table 4 Comparative data of storage stability between aclonifen SC samples

| Sample | 1 month | | 3 months | | 6 months | |
|---|---|---|---|---|---|---|
| | Concentration of aclonifen (%) | Aggregation | Concentration of aclonifen (%) | Aggregation | Concentration of aclonifen (%) | Aggregation |
| Example 4 | 59 | - | 58 | + | 56 | +++ |
| Example 5 | 60 | - | 60 | - | 60 | - |

In Table 4 above, "+" indicates a small amount of aggregation, "+++" indicates a large amount of aggregation, and "-" indicates no aggregation.

The above examples are preferred examples of the present disclosure rather than limitations to the present disclosure in any form and substance. It should be noted that those of ordinary skill in the art may make various improvements and supplementations without departing from the principles of the present disclosure, and these improvements and supplementations should be regarded as falling within the protection scope of the present disclosure.

## Claims

**1.** A crystal form of aclonifen, wherein the crystal form is denoted as an aclonifen solid form A; and the crystal form presents the following reflections at 2θ±0.20° in any combination in an X-ray powder diffraction (X-RPD) pattern recorded at 25°C using Cu-Ka radiation:

$$2\theta = 9.94 \pm 0.20 \quad (1)$$

$$2\theta = 10.98 \pm 0.20 \quad (2)$$

$$2\theta = 14.72 \pm 0.20 \quad (3)$$

$$2\theta = 15.08 \pm 0.20 \quad (4)$$

$$2\theta = 15.48 \pm 0.20 \quad (5)$$

$$2\theta = 19.26 \pm 0.20 \quad (6)$$

$$2\theta = 20.82 \pm 0.20 \quad (7)$$

$$2\theta = 22.74 \pm 0.20 \quad (8)$$

$$2\theta = 24.20 \pm 0.20 \quad (9)$$

$$2\theta = 25.04 \pm 0.20 \quad (10)$$

$$2\theta = 27.28 \pm 0.20 \quad (11)$$

$$2\theta = 28.20 \pm 0.20 \quad (12).$$

2. The aclonifen solid form A according to claim 1, wherein the aclonifen solid form A has an infrared (IR) spectrum with a characteristic functional group vibration peak at one or more wavenumbers ($cm^{-1}$, ±0.2%) of 3,503 $cm^{-1}$, 3,390 $cm^{-1}$, 1,621 $cm^{-1}$, 1,591 $cm^{-1}$, 1,568 $cm^{-1}$, 1,551 $cm^{-1}$, 1,485 $cm^{-1}$, 1,462 $cm^{-1}$, 1,421 $cm^{-1}$, 1,357 $cm^{-1}$, 1,243 $cm^{-1}$, 1,112 $cm^{-1}$, and 754 $cm^{-1}$; and/or
the aclonifen solid form A has a differential scanning calorimetry (DSC) curve with an endothermic melting peak at 82.5°C.

3. A method for preparing the aclonifen solid form A according to claim 1 or 2, comprising the following steps:

   step 1, dissolving aclonifen in a solvent, wherein the aclonifen is amorphous aclonifen;
   step 2, precipitating dissolved aclonifen to produce the aclonifen solid form A; and
   step 3, separating the precipitated aclonifen solid form A.

4. The method according to claim 3, wherein in the step 1, the solvent is selected from at least one of methanol and ethanol, and the dissolving is conducted by heating.

5. The method according to claim 3, wherein in the step 2, the precipitating dissolved aclonifen to produce the aclonifen solid form A specifically comprises: concentrating a solution and/or cooling and/or adding a solvent for reducing solubility and/or adding a crystal seed of the aclonifen solid form A.

6. The method according to claim 3, wherein the step 2 is implemented by cooling to 0°C to 15°C.

7. An aclonifen solid form A prepared by the method according to claim 3, wherein the aclonifen solid form A has an aclonifen solid form A content of at least 97% in a weight percentage.

8. A composition, comprising the aclonifen solid form A according to any one of claims 1, 2, and 7 and at least one additive, wherein the additive is selected from one or more of the following: a surfactant, a diluent, a dispersing agent, a wetting agent, an antioxidant, a defoaming agent, an antifreeze agent, and a thickening agent.

9. The composition according to claim 8, wherein the composition comprises the aclonifen solid form A of less than 80% in a weight percentage.

10. A use of the aclonifen solid form A according to any one of claims 1, 2, and 7 or the composition according to claim 8 in preparation of an herbicide.

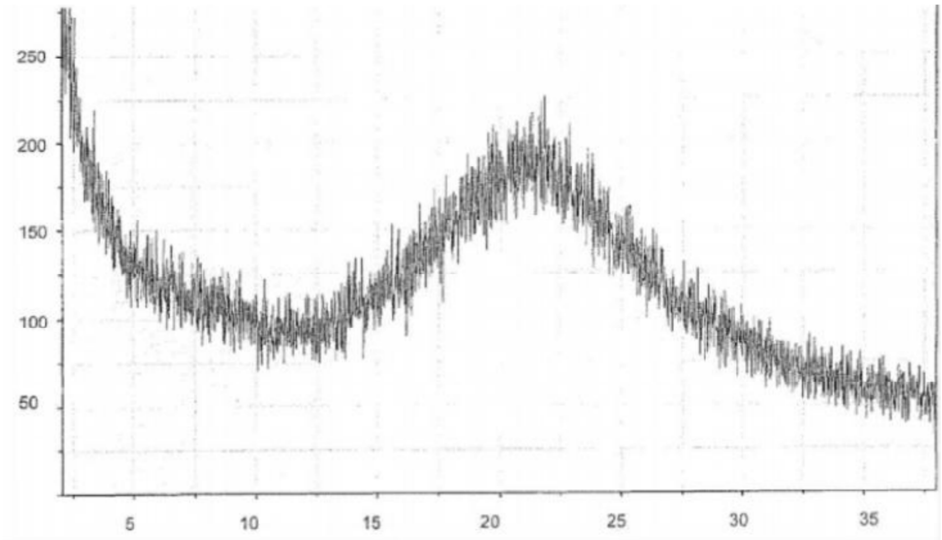
250
200
150
100
50
5
10
15
20
25
30
35

FIG. 1

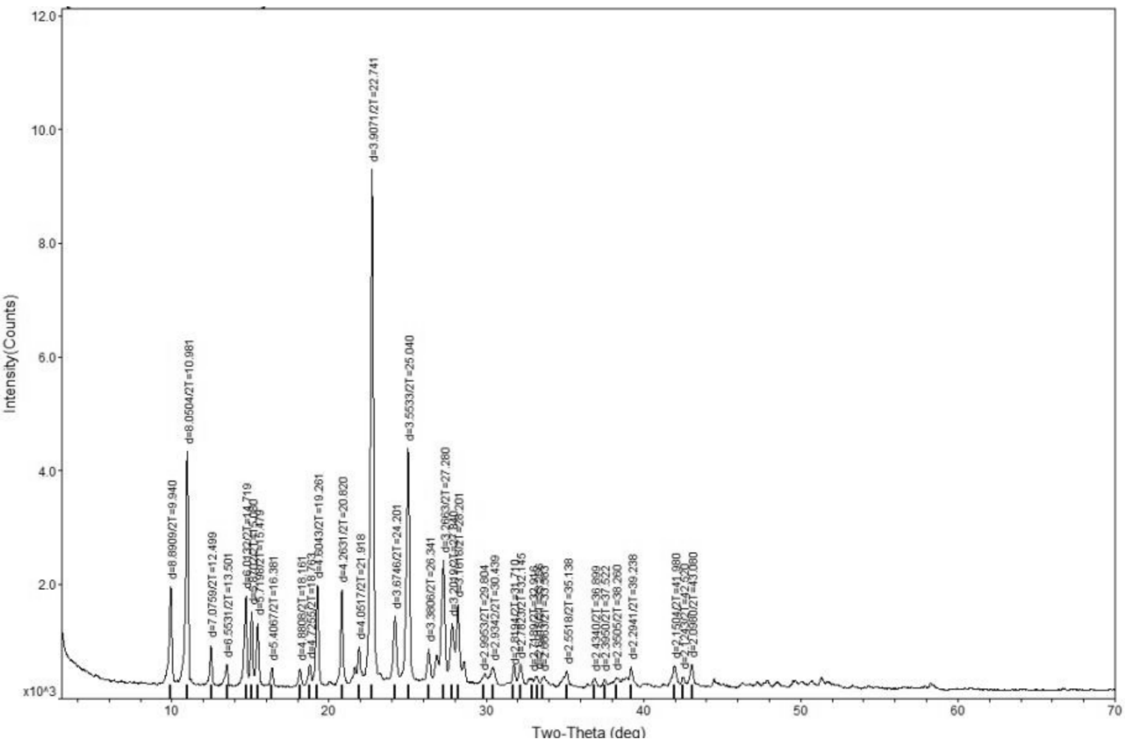
12.0
10.0
8.0
6.0
4.0
2.0
x10^3
Intensity(Counts)
10
20
30
40
50
60
70
Two-Theta (deg)

FIG. 2

Analyst Administrator PerkinElmer Spectrum version 10.5.4
Date 14: 18 on January 15, 2024 14: 18 on January 15, 2024

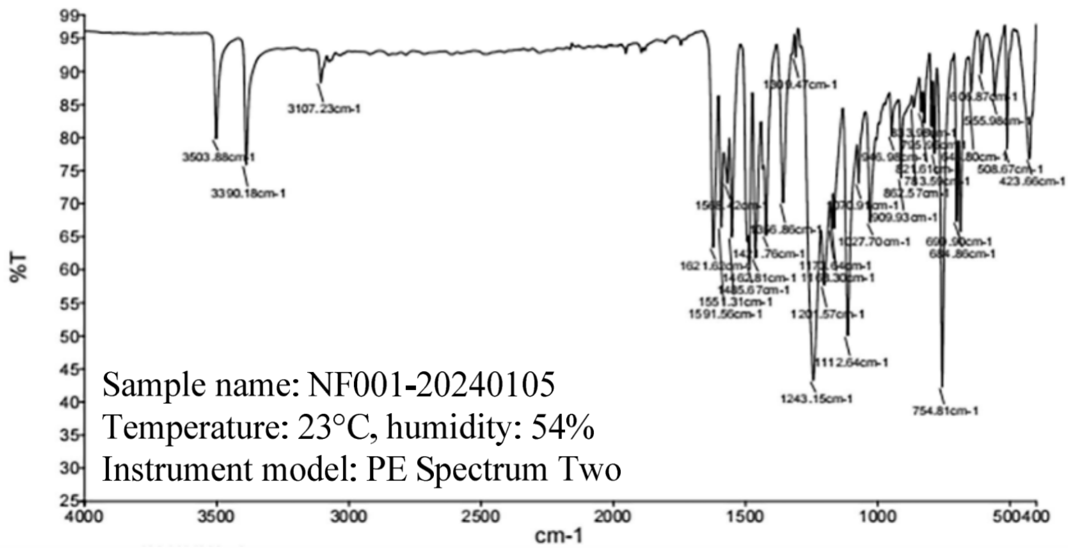


FIG. 3

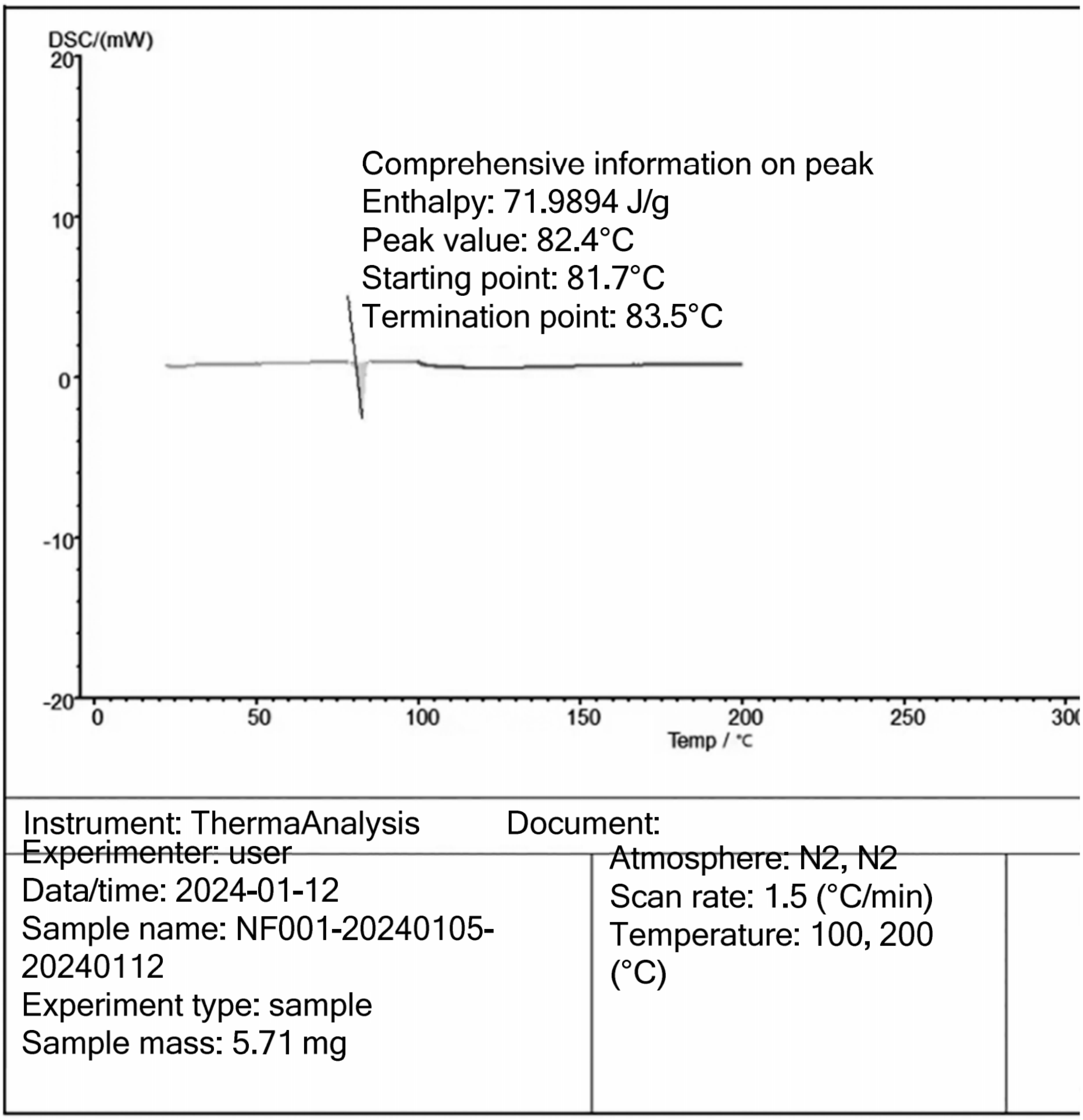
DSC/(mW)
20
10
0
-10
-20
0
50
100
150
200
250
300
Temp / °C
Comprehensive information on peak
Enthalpy: 71.9894 J/g
Peak value: 82.4°C
Starting point: 81.7°C
Termination point: 83.5°C
Instrument: ThermaAnalysis
Document:
Experimenter: user
Data/time: 2024-01-12
Sample name: NF001-20240105-20240112
Experiment type: sample
Sample mass: 5.71 mg
Atmosphere: N2, N2
Scan rate: 1.5 (°C/min)
Temperature: 100, 200 (°C)

FIG. 4

**INTERNATIONAL SEARCH REPORT**

International application No.
**PCT/CN2024/087918**

**A. CLASSIFICATION OF SUBJECT MATTER**

C07C217/90(2006.01)i

According to International Patent Classification (IPC) or to both national classification and IPC

**B. FIELDS SEARCHED**

Minimum documentation searched (classification system followed by classification symbols)

IPC: C07C 217

Documentation searched other than minimum documentation to the extent that such documents are included in the fields searched

Electronic data base consulted during the international search (name of data base and, where practicable, search terms used)

CNTXT, DWPI, ENTXT, ENTXTC, CNKI, 读秀, DUXIU, Web of Science, STN(CAplus): 苯胺, 苯草醚, 苯氧基, 氯, 硝基, 结晶, 晶型, 晶, 稳定, aclonifen, benzenamine, chloro, nitro, phenoxy, aniline, crystal, 74070-46-5/RN

**C. DOCUMENTS CONSIDERED TO BE RELEVANT**

| Category* | Citation of document, with indication, where appropriate, of the relevant passages | Relevant to claim No. |
|---|---|---|
| A | CN 115108922 A (SHANGHAI NONGFAN BIOTECHNOLOGY CO., LTD.) 27 September 2022 (2022-09-27)<br>claims 1 and 4, and description, embodiment 1 | 1-10 |
| A | WO 2023183353 A2 (FMC CORP.) 28 September 2023 (2023-09-28)<br>claims 1 and 10, and description, paragraphs 6, 9, 50, and 205 | 1-10 |
| A | US 4394159 A (CELAMERCK GMBH & CO. KG) 19 July 1983 (1983-07-19)<br>claim 2, and description, column 6, paragraph 2, embodiment 23 | 1-10 |

☐ Further documents are listed in the continuation of Box C.  ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **10 December 2024** | **25 December 2024** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**
**Information on patent family members**

International application No.
**PCT/CN2024/087918**

| Patent document cited in search report | Publication date (day/month/year) | Patent family member(s) | Publication date (day/month/year) |
|---|---|---|---|
| CN 115108922 A | 27 September 2022 | CN 115108922 B | 28 June 2024 |
| WO 2023183353 A2 | 28 September 2023 | TW 202345697 A | 01 December 2023 |
| | | MX 2024011481 A | 26 September 2024 |
| | | AU 2023240976 A1 | 19 September 2024 |
| | | WO 2023183353 A3 | 02 November 2023 |
| | | IL 315169 A | 01 October 2024 |
| | | AR 128831 A1 | 19 June 2024 |
| US 4394159 A | 19 July 1983 | BR 7904341 A | 08 April 1980 |
| | | HU 182935 B | 28 March 1984 |
| | | CS 213387 B2 | 09 April 1982 |
| | | IN 152539 B | 04 February 1984 |
| | | DE 2831262 A1 | 31 January 1980 |
| | | ES 482498 A0 | 01 December 1980 |
| | | NZ 191009 A | 23 March 1982 |
| | | GR 75066 B | 13 July 1984 |
| | | MA 18525 A1 | 01 April 1980 |
| | | DK 297179 A | 16 January 1980 |
| | | DK 157750 B | 12 February 1990 |
| | | DK 157750 C | 09 July 1990 |
| | | PL 217051 A1 | 14 July 1980 |
| | | PL 121517 B1 | 31 May 1982 |
| | | YU 170479 A | 28 February 1983 |
| | | YU 42191 B | 30 June 1988 |
| | | EG 14414 A | 31 December 1983 |
| | | CA 1108169 A | 01 September 1981 |
| | | TR 20258 A | 08 December 1980 |
| | | BG 41301 A3 | 15 May 1987 |
| | | SU 882402 A3 | 15 November 1981 |
| | | ZA 793543 B | 25 March 1981 |
| | | RO 78105 A | 01 February 1982 |
| | | ATA 475879 A | 15 November 1981 |
| | | AT 366881 B | 10 May 1982 |
| | | ZA 7903543 B | 25 March 1981 |
| | | EP 0007482 A1 | 06 February 1980 |
| | | EP 0007482 B1 | 16 September 1981 |
| | | DE 2960829 D1 | 03 December 1981 |
| | | ES 482497 A1 | 16 May 1980 |
| | | ES 8107157 A1 | 01 December 1980 |
| | | USRE 31717 E | 30 October 1984 |
| | | AR 221244 A1 | 15 January 1981 |
| | | IL 57794 A0 | 30 November 1979 |
| | | IL 57794 A | 30 November 1982 |
| | | DD 146177 A5 | 28 January 1981 |
| | | JPS 5519260 A | 09 February 1980 |
| | | JPS 6259692 B2 | 12 December 1987 |
| | | AU 4891079 A | 24 January 1980 |
| | | AU 521242 B2 | 25 March 1982 |
| | | MX 5578 E | 19 October 1983 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 4394159 A